# EUROPEAN PATENT APPLICATION

(11) **EP 0 694 616 A2**
(43) Date of publication of application: **31.01.1996**
(21) Application number: 95111587.2
(22) Date of filing: 24.07.1995
(51) Int. Cl.: C12P 19/26, C12N 1/20

(54) **Process for the preparation of hyaluronic acid by fermentation with streptococcus**

(30) Priority: 26.07.1994 IT MI941594
(71) Applicant: POLI INDUSTRIA CHIMICA S.p.A., I-20141 Milano (IT)
(72) Inventor: Poli, Stefano, I-20089 Quinto de' Stampi-Rozzano (MI) (IT); Bocchiola, Gianettore, I-20089 Quinto de' Stampi-Rozzano (MI) (IT); Casareto, Enrico, I-20089 Quinto de' Stampi-Rozzano (MI) (IT); Leoni, Massimo, I-20089 Quinto de' Stampi-Rozzano (MI) (IT); Magni, Ambrogio, I-20089 Quinto de' Stampi-Rozzano (MI) (IT); Ronzio, Enrico, I-20089 Quinto de' Stampi-Rozzano (MI) (IT)
(74) Representative: Minoja, Fabrizio

(57) **Abstract**

A process for the preparation of hyaluronic acid by submerged culture of a microorganism of the genus Streptococcus is described.

## Description

The present invention relates to a process for the preparation of hyaluronic acid by submerged culture of a microorganism of the genus Streptococcus.

### State of the art and technical problem

Hyaluronic acid is a macromolecule consisting of N-acetylglucosamine and glucuronic acid present in equimolar ratio and has molecular weights variable from a few tens of thousands to several million Dalton (D), depending on its source. In fact, hyaluronic acid is a substance which is widely distributed in nature, in particular in higher organisms, inasmuch as it is one of the components of the extracellular matrix of the connective tissues of animals. It is also abundant in the umbilical cord, in the eye, in the joint capsules of mammals, in cartilages and in the crests of gallinaceous birds. Nevertheless, the limited availability of these materials, together with the fact that the products of natural origin are very often obtained in a state contaminated with proteins, and also the difficulty of extraction and the rather low yields have stimulated a search for alternative sources which can provide a purer product of higher molecular weight at lower production costs owing to a higher yield of the process and a greater ease of extraction and purification.

The ability of some bacterial species to produce hyaluronic acid has been described in the past. Almost all the microorganisms having this property belong to the genus Streptococcus (S. equi, S. equisimilis, S. zooepidemicus, S. dysgalactiae, S. pyogenes) or to the genus Pasteurella (P. multocida) or to the genus Pseudomonas (P. aeruginosa).

S. zooepidemicus in the same way as S. equi, the other microorganism most frequently used for the industrial production of hyaluronic acid, belongs to group C of the Lancefield classification which includes strains usually associated with infections in animals; the use of these strains in a production process involves fewer risks than the use of strains belonging to Lancefield group A, such as S. pyogenes.

The Streptococci are capable of producing the characteristic phenomenon of haemolysis, which is common to a large number of pathogenic microorganisms.

This phenomenon involves the fact that the hyaluronic acid produced can be contaminated with β-haemolysin, and such contamination makes the use of a product which has not been carefully purified extremely dangerous.

Like the production of haemolysin, the biosynthesis of hyaluronidase is also a constant characteristic of the virulent pathogenic Streptococcus strains, which is the cause of a further problem, namely the degradation of the desired product.

From the point of view of the nutritional requirements, the pathogenic Streptococci are to be counted amongst the most demanding microorganisms. For this reason, the strains are maintained on complex media such as, for example, brain heart infusion broth agar or tryptic soy broth agar (BHIA, TSA) based on extracts or infusions of organs such as ox brain or heart. It is clear that the fermentation medium must also contain, in addition to suitable sources of carbon and mineral salts, complex raw materials which can provide the right amount of those amino acids, vitamins, purine bases and pyrimidine bases which the microorganism is incapable of synthesizing autonomously: an abundant development of biomass is in fact an indispensable premise for good production of hyaluronic acid.

The principal carbon source is generally glucose, fructose, raffinose or mannitol in concentrations from 10 to 100 grams/litre, to which organic acids such as acetic acid, aspartic acid, glutamic acid, malic acid or oxaloacetic acid can be added in concentrations from 1 to 20 g/l, preferably from 5 to 10 g/l. When the carbohydrates are used in large quantities, they can be added gradually as a concentrated solution in the course of the fermentation. The choice of when to carry out complementary addition of carbohydrates is of crucial importance to good growth of the microorganism and to good production of hyaluronic acid.

The metabolism of the sugars involves the production of acidic substances (principally lactic acid and acetic acid), whose accumulation lowers the pH fairly quickly to such values that the growth of the microorganism is inhibited.

The importance of the role played by the complex raw materials in the hyaluronic acid fermentation with S. zooepidemicus has already been pointed out. Among these, we mention extracts of animal origin, such as meat extract and protein extract from blood, or of microbial origin, such as yeast extract, as those extracts which can be used advantageously for the culturing. Equally, lysates or hydrolysates of various origins, such as yeast autolysate, corn steep and casein hydrolysate, or peptones such as the peptones from blood, meat, liver, soya, casein and skimmed milk give very good results. The optimum concentration of these substances varies according to the strain used, but it is generally between 5 and 50 g/l. Where substances containing thermolabile principles are concerned, heating can reduce, sometimes drastically, the nutritive quality of the medium. For this reason, the sterilization profile of the fermenters must be carefully studied in order to determine the minimum quantity of heat to be transferred to the medium without appreciably diminishing the guarantee of being able to conduct a perfectly axenic process.

An extremely useful approach aimed at improving a fermentation process is the study of the metabolic pathways which lead from the substrates to the final product which it is desired to produce; that is to say, in order to initiate the phase of "medium improvement", in which an evaluation is carried out of the substrates or the nutritional elements capable of promoting substantial improvements in the yield of the useful substances in the biosynthetic process without, on the other hand, depressing other metabolic pathways indispensable to the life of the microorganism.

Normally, the inactivation of the microorganism at the end of the fermentation is carried out by addition of acids to the broth culture; generally, trichloroacetic acid is preferred for these operations. It has been found that the use of this acid on an industrial scale has the following disadvantages:
1) significant problems in the ecological breakdown of the wastes containing this acid, due to the high chlorine content,
2) a notable degradation of the mean molecular weight from the value measured at the end of the fermentation and that found after the isolation of the product has been observed; this phenomenon is the more marked the longer the time and the higher the working temperature.

The principal techniques hitherto used for the recovery and purification of hyaluronic acid, which can have an effective industrial application, are:
- ultrafiltration used either for removing impurities of low molecular weight or for effecting fractionations of the product on the basis of the molecular weight (see, for example, WO 92/18,543 and JP 63-094,988)
- chromatography on ion exchange resins, where normally the hyaluronic acid is fixed on the resin in order to enable the accompanying impurities to be removed, and then the product is liberated by elution with buffers, in order to effect a selective displacement (see, for example, JP 63289198). In some cases, the purification of hyaluronic acid has been described, where only a part thereof having a molecular weight of ≦ 2,000,000 D is retained on the resin (see, for example, WO 89/06,243)
- precipitation of the hyaluronic acid as quaternary ammonium salt, its isolation by filtration and subsequent redissolution of the salt in a solution which, by addition of Na⁺ ions, again forms sodium hyaluronate (see, for example, JP 2-947,101 and JP 63-270,701).

When the final purification of hyaluronic acid is carried out by ion exchange chromatography, the following drawbacks arise:
1 the use of reagents for the isolation, which must then be separated from the hyaluronic acid for further purification, such as, for example, precipitation with solvents,
2 during the isolation of the hyaluronic acid, there is a significant change in the viscosity of the eluted solution, which leads to a reduction in flow and to a not inconsiderable increase in the pressure in the column,
3 the elution of the hyaluronic acid at levels of quantitative interest leads to a lowering of its concentration, which then requires considerable quantities of solvent for the precipitation.

Summarizing the state of the art, the current problems which arise for an expert in the field of the preparation of hyaluronic acid by the microbiological route, in particular by the use of microorganisms of the genus Streptococcus zooepidemicus and Streptococcus equi, are therefore: contamination with β-haemolysin and hyaluronidase; production of hyaluronic acid of high molecular weight and free of by-products derived from its degradation; optimization of the culture medium as a function of the quantitative yield of hyaluronic acid; production of a final product under pharmaceutically acceptable conditions or alternatively assurance of a sterile and non-pyrogenic product; a purification phase which is still laborious.

These problems have been solved by the present invention which provides a process for the preparation of hyaluronic acid by means of a submerged culture of a microorganism of the genus Streptococcus zooepidemicus or Streptococcus equi. The hyaluronic acid obtained by the process of the present invention is obtained free of β-haemolysin, is stable and has a high molecular weight and high purity.

### Summary of the invention

A subject of the present invention is a process for the preparation of hyaluronic acid, comprising the culturing of a microorganism of the genus Streptococcus zooepidemicus or Streptococcus equi, the inactivation of the microorganism and the isolation of said hyaluronic acid, characterized in that the culturing of the microorganism is carried out in the presence of divalent iron ions.

Moreover, a strain of Streptococcus zooepidemicus has been isolated which was obtained by the Applicant after repeated cycles of mutagenesis and selection and which has been used in the process of the present invention, and which is capable of producing very high yields of hyaluronic acid which, apart from being free of β-haemolysin, is characterized by a high molecular weight.

The mutated strain, which is designated UV1 here, was deposited at CNCM on 21 July 1994 under the accession number I-1448 and is a further subject of the present invention.

A further subject of the present invention is the abovementioned process which comprises the culturing of the strain UV1.

### Detailed description of the invention

According to the present invention, the process for preparation of hyaluronic acid is carried out by culturing a microorganism, namely Streptococcus zooepidemicus or Streptococcus equi, which can be acquired from a collection.

The culturing is carried out according to the conventional technique of submerged culture in a nutrient medium which is suitable for this type of microorganism and whose composition is known to those skilled in the field.

It has been found, surprisingly, that the addition of ferrous ions to the culture medium inhibits the production of β-haemolysin. Therefore, according to the present invention, inorganic salts of iron, such as sulphites, sulphates, chlorides, fluorides, nitrates, chlorates or phosphates, are added to the fermentation medium in such a quantity that there are 1-100 mg/l, preferably 5-50 mg/l, of ferrous (Fe⁺⁺) ions in solution. Such concentrations are surprisingly capable of repressing the expression mechanisms of the specific iron uptake systems of S. zooepidemicus, specifically the synthesis of β-haemolysin. In this way, it becomes possible to obtain a product free of β-haemolysin contaminants, to the great advantage of the purification process and of the final quality of the hyaluronic acid.

Surprisingly, in the presence of an excess of Fe⁺⁺ ions, at the end of fermentation the broth, apart from being free of haemolysin, contains hyaluronic acid of a higher molecular weight than that obtainable in a medium which is the same except that it lacks iron. The presence of an excess of ferrous ions in the medium, together with the precaution of constantly monitoring the viscosity of the fermentation broth, makes it possible to optimize both the quantity and the molecular weight of the hyaluronic acid produced.

In a first, preferred embodiment, the process according to the present invention is carried out using the strain UV1 of Streptococcus zooepidemicus.

Said strain has been obtained by the Applicant by mutagenesis of a wild strain.

Some biochemical characteristics of the strain UV1 are listed below:

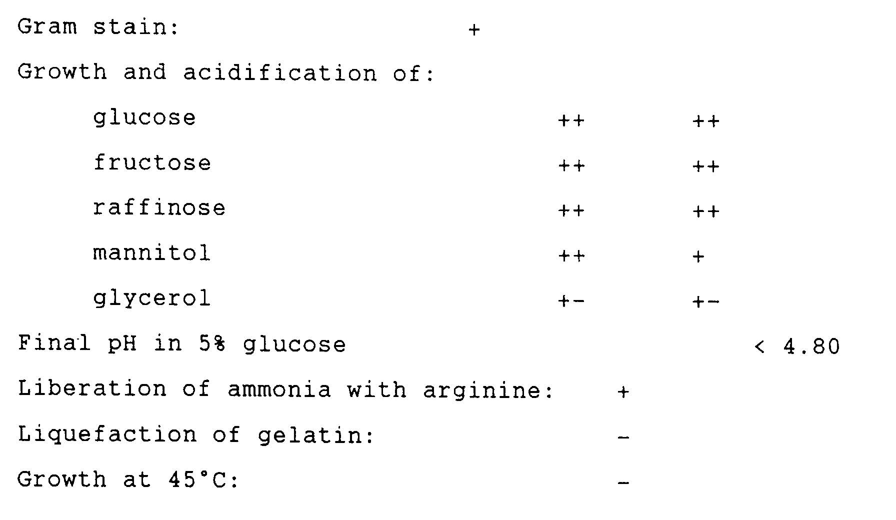

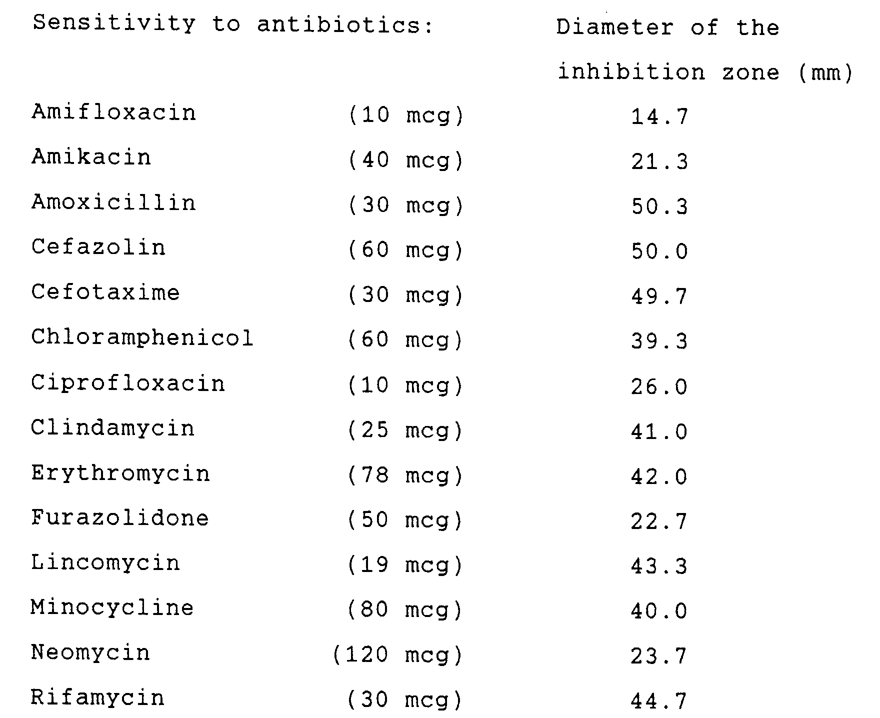

The UV1 strain of S. zooepidemicus grows rapidly on complex liquid media such as brain heart infusion broth (BHIB) or tryptic soy broth (TSB) and on their corresponding agar media (BHIA and TSA). On these solid media, the strain shows, after 24-36 hours of growth at a temperature of 30°C, circular, translucent colonies of a more viscous consistency than that of the normal unmutated strain: the mutation is therefore demonstrated in the sense of a higher productivity of hyaluronic acid.

The strain described here shows good development (within 24 hours at 30°C) on a solid agar-blood medium, where the characteristic β-haemolytic reaction of the Streptococci is displayed in a very clear manner.

According to a second embodiment, the process which is the subject of the present invention provides, with the aim of optimizing the production of biomass, a particular cycle of fractionated addition of carbohydrates in 3 parts, which is to be carried out, respectively, at the moment of inoculation, half an hour after the minimum level of dissolved oxygen has been reached and 6 hours after the second addition, this cycle being capable of maximizing the production of biomass and hence of hyaluronic acid.

Advantageously, the pH of the broth culture is continuously controlled, maintaining it in the range of 6.5 ± 1.0 units, preferably between 6.5 and 7.5 units. The correction is usually carried out by means of alkali concentrated aqueous solutions, such as sodium or potassium hydroxide, and can also be effected during the vegetative phase. Alternatively, it is also possible to use powdered calcium carbonate or other known methods.

This expedient adopted not only in the course of the fermentation, but also in the course of the vegetative culturing stages (seed) which precede the inoculation of the production fermenter, makes it possible to arrange for there to be inocula of perfect vitality in the phase of exponential growth, with high biomass content and with a consequent shortening of the lag phase in the following fermentative stage.

A further procedural measure, which has led to unexpected results in terms of growth of the biomass then consists of raising the overpressure in the fermenter to values higher than 0.1 bar up to 3.5 bar. Without wishing to be tied to any theoretical consideration, one possible, but not exhaustive, explanation of this phenomenon can be sought in the solubility of CO₂ in the aqueous media: because the growth of the microorganism takes place in submerged culture in the hyaluronic acid production process, the medium is subjected to forced and continuous aeration which brings about constant removal of the CO₂ produced by the bacteria, and it is then assumed that the overpressure makes it possible to increase the amount of this gas dissolved in the aqueous medium.

The problem of the sterilization of the nutrient medium before the inoculation also arises. This phase is important as regards the yield, since many nutrient substances are thermolabile.

According to the present invention, a sterilization cycle carried out without exceeding 120°C, for a maximum period of 30 minutes, leads to totally unexpected results in terms of yield.

The present invention also faced the problem of the optimization of the culture medium.

From this point of view, it was, surprisingly, found to be useful to raise the concentration of UTP in the intracellular pool, which is achievable by means of the addition of uracil to the fermentation medium, and equally good results have, surprisingly, been produced by the addition of glutamine and aspartate to the medium. We have been able effectively to establish that, with the addition of the three molecules specified above (uracil, aspartic acid and glutamine) to the fermentation medium of S. zooepidemicus, preferably the UV1 strain, yields are obtained in the production of hyaluronic acid which are 20-25% higher than those obtainable in the absence thereof. The useful concentrations of the three substrates for attaining such a result are between 0.01 and 10 g/l, preferably between 0.5 and 3 g/l.

It is known that the inactivation of the microorganism is effected by adding an acid to the culture medium.

As mentioned above, the conventional addition of trichloroacetic acid involves problems with waste treatment and the integrity of the final product.

In an attempt to overcome these problems, various acids were used for carrying out the inactivation of the microorganism, such as sulphuric acid, phosphoric acid, hypophosphorous acid, lactic acid and tartaric acid; among these, hypophosphorous acid added until a pH below 4.8 is reached has proved to be the most advantageous as regards maintaining the value of the mean molecular weight.

Surprisingly, it has been confirmed that, after sterilization of the fermentation broth, in an attempt to remove the cells of the microorganism from the solution containing the hyaluronic acid by tangential filtration on a microfiltration membrane, the desired product did not permeate the latter, but the sodium hyaluronate solution was concentrated with the cells. The microfiltration operations were carried out with membranes of the polymeric or ceramic type and of sintered metal, having a porosity of between 0.05 and 5 µm.

This unforeseen capacity of the microfiltration membranes to retain the hyaluronic acid and its salts represents a great advantage in terms of process economics, since it makes it possible to obtain extremely pure solutions of hyaluronic acid at reduced relative operating costs, which can be achieved with fairly modest equipment.

In this way, a product with more than 95% of dry matter with a content of residual protein of ≦ 0.3% is obtained after isolation.

In another experimentation series, it has been found that the hyaluronic acid is purified, by removal of proteins and other accompanying substances, by combined passage through a first bed of ion exchange resin in the cationic H⁺ form and subsequently a bed of resin in the anionic form. The novel phenomenon, which was not foreseeable in this experimentation, is that hyaluronic acid of any molecular weight is not fixed on the resin, but passes unaltered through both beds, while the impurities remain fixed, such as the proteins which are predominantly retained on the resin in the H⁺ form. The effect is so marked that the treatment can also be carried out not in a column, but in a stirred batch reactor.

According to a further embodiment, the solid product can be obtained directly from the broth culture solutions without any isolation therefrom up to drying, which occurs by the processes of lyophilization, atomization or exposure to heat under reduced pressure.

From the solutions obtained either by chromatography or by microfiltration, the hyaluronic acid can be recovered directly as sodium salt by means of lyophilization or by subjecting the solution to the action of a spray dryer; the sodium hyaluronate obtained under these conditions is sufficiently pure to be marketed commercially. If it is desired to obtain sodium hyaluronate of high purity (> 98%), the solution is concentrated by microfiltration until a solution between 1 and 2% is obtained from which the sodium hyaluronate is precipitated with solvents such as ethanol, isopropanol and acetone, the formation of the precipitate being assisted by the addition of sodium salts such as the chloride, sulphate, phosphate and preferably acetate or other anions of organic acids.

Between the preparation of solutions of hyaluronic acid and their treatment for recovery of the purified product, a significant reduction in the mean molecular weight (from 1,500,000 D to about 600,000 D) is encountered, together with an increase in the turbidity of the solutions; the addition of alcoholic or ketonic solvents or of substances containing a phenolic functional group or of ionic and nonionic detergent substances is capable of preventing this phenomenon by keeping the solutions of hyaluronic acid clear and free of bacterial contaminations over time.

The high degree of purity of the hyaluronic acid obtained by the process described makes it an ideal candidate for use in products of the pharmaceutical and/or cosmetic type with superior quality. The method claimed makes it possible to obtain a product which, as has been verified, is totally free from tolerability problems, and its use, while highly effective at a general level, proves to be particularly advantageous for the treatment of hypersensitive areas such as the vaginal, nasal, ocular etc. mucous membranes.

The following examples further illustrate the invention.

### EXAMPLE 1

A medium prepared with:
30 g/l dextrose,
10 g/l peptone,
10 g/l yeast extract,
10 g/l monobasic potassium phosphate,
7 g/l sodium acetate,
5 g/l sodium bicarbonate,
10 mg/l ferrous sulphate,
300 mg/l magnesium sulphate,
8 mg/l manganese sulphate,
80 mg/l calcium chloride and
100 mg/l antifoam,
is adjusted to a pH of 7.00 by addition of NaOH and is then sterilized for 20 minutes at 121°C. The inoculation employs a concentration of 2.5% of a culture of Streptococcus zooepidemicus obtained from a collection. The fermentation takes about 24 hours at 37°C, with the pH maintained at 7.00 by continuous automatic addition of NaOH. 33% of the dextrose is added at the moment of inoculation, and the remainder is administered continuously. The yield of the process determined by HPLC analysis is 0.8 g/l with a mean molecular weight estimated at around 500,000 D.

### EXAMPLE 2

Following the procedure described in the preceding example, a fermentation is carried out with the only difference being that the inoculation takes place with mutated and selected Streptococcus zooepidemicus designated UV1. The yield of the process determined as above is 1.5 g/l with a mean molecular weight of 800,000 D.

### EXAMPLE 3

The fermentation is carried out as described in the preceding example, but the quantity of ferrous sulphate is tripled by means of two additions after 8 and 16 hours. The yield of the process is 1.4 g/l, but with a mean molecular weight of 1.5-2.5 million D. The final broth also proves to be free of streptolysin.

### EXAMPLE 4

The procedure of the preceding example is followed, but the inoculum is maintained at pH 7.00 by continuous addition of NaOH. This allows the biomass of the inoculum to be doubled. The entire fermentation is accelerated and the yield after 20 hours of fermentation, at equal mean molecular weight, is 1.8 g/l.

### EXAMPLE 5

The procedure of the preceding example is followed, but the fermentation is carried out, where a overpressure of 0.6 bar is maintained. The yield, at equal molecular weight, increases by about 10%.

### EXAMPLE 6

The fermentation is carried out by the procedure described in the preceding example, but the thermal treatment of the culture broth is reduced to 10 minutes at 110°C in order to preserve the fertility thereof. The yield is 2.8 g/l, at equal molecular weight.

### EXAMPLE 7

The influence of some metabolic precursors on the fermentation is investigated: the same inoculum is subdivided into 4 fermenters operated as in the preceding example, but respectively 2 g/l uracil, 2 g/l aspartic acid, 2 g/l glutamine or simultaneously the 3 precursors at the same concentration of 2 g/l are added to the initial medium. The results obtained, always at equal mean molecular weight, are as follows:

| | | |
|---|---|---|
| a) | initial medium + uracil | 3.3 g/l |
| b) | initial medium + aspartic acid | 3.0 g/l |
| c) | initial medium + glutamine | 2.9 g/l. |

Small improvements (2-3%) are obtained by addition of all three of the precursors.

### EXAMPLE 8

The procedure of the preceding example (7a) is followed, but the glucose is added at a rate of one third at the moment of inoculation, one third half an hour after the condition of anaerobiosis of the culture has been reached, and one third 5-6 hours after the second addition. The fermentation yield is 3.5 g/l at a mean molecular weight of > 2 million D.

### EXAMPLE 9

The bacterium of the broth culture obtained under the conditions of Example 8 is inactivated by lowering the pH down to 3.5 by addition of hypophosphorous acid. The disappearance of the activity of the Streptococcus is evaluated by means of a plate test after reestablishing a neutral pH.

For this purpose, various organic and inorganic acids were tested. The table which follows gives indications regarding the efficiency of the inactivation and the influence on the lysis of the polymer by means of a viscosity measurement.

| Acid | % reduction in the viscosity of the broth | Inactivity of the microorganism |
|---|---|---|
| Sulphuric | 44.3 | complete |
| Trichloroacetic | 42.9 | complete |
| Phosphoric | 37.7 | complete |
| Hypophosphorous | 23.2 | complete |
| Lactic | 35.7 | complete |
| Tartaric | 32.5 | partial |

### EXAMPLE 10

a) From a part of the fermentation broth acidified to pH 3.5 with hypophosphorous acid and containing 5 g of hyaluronic acid with added filter aid, the insoluble matter is removed by filtration and washing of the filter with water. Acetone is added to the filtrate in order to induce the precipitation of the crude product which, after removal of the mother liquors, is redissolved in water up to complete solution in the presence of 5% of acetone. The solution thus obtained is passed through a column charged with strong cationic resin IMAC C16P in the H⁺ form. The solution thus treated is made to pass through a second column charged with strong anionic resin IRA 900 in the OH form. 20 g of sodium acetate are added to the solution which is warmed to 40°C, and ethanol is then added in order to obtain the precipitation of the hyaluronic acid as sodium salt. The precipitate is filtered off, washed with ethanol and dried under reduced pressure, to give 4 g of product with the following quality profile:
   - content of dry product, about 98%
   - content of residual proteins about 0.1% (haemolysin test: negative)
   - mean molecular weight greater than 1,500,000 D
   - the transmittance of a 0.2% solution is 99.8%.
b) When treating a solution containing 5 g of crude hyaluronic acid of mean molecular weight < 200,000 D under the same conditions as in Example 10 a), 4.3 g of hyaluronic acid having the following quality profile are obtained:
   - content of dry product, about 98%
   - content of residual proteins 0.05%
   - mean molecular weight 180,000 D
   - transmittance of a 0.2% solution 95%.

### EXAMPLE 11

The test of Example 10 a) is repeated, omitting the addition of 5% of acetone to the solution of the product before the treatment on the resins: this gives 4 g of hyaluronic acid having the following quality profile:
- content of dry product, about 98%
- content of residual proteins 0.1%
- mean molecular weight 650,000 D
- transmittance of a 0.2% solution 95%.

### EXAMPLE 12

After the crude product has been precipitated under the conditions described in Example 10, it is redissolved in water containing 10% of acetone. The solution thus obtained is subjected to a tangential filtration on a microfiltration membrane, taking care to maintain a high linear velocity in the filtration loop but controlling it in order to give a large permeation flow. There is an outflow across the membrane of a permeate which contains proteins and mineral salts and is free of hyaluronic acid. Water is continuously fed to the mixture to be purified at such a rate that the initial volume is restored, until the resulting permeate is free of residues. The solution is then concentrated up to about 1% before proceeding with the isolation of the product as in Example 2. The quality specifications are as follows:
- content of dry product > 96%
- content of proteins about 0.1%
- mean molecular weight > 1,500,000 D
- transmittance of a 0.2% solution > 99.5%

### EXAMPLE 13

The test under the conditions of Example 12 is repeated, omitting the addition of solvent to the solution before the filtration. This gives a product having the following quality specifications:
- content of dry product > 96%
- content of proteins about 0.1%
- mean molecular weight about 600,000 D
- transmittance of a 0.2% solution 91%

### EXAMPLE 14

The test under the conditions of Example 10 is repeated, with the difference that the solution treated with the two resins is subjected to lyophilization on a plate, freezing at -40°C for 4 hours and using a very slow lyophilization cycle up to a maximum warming temperature of 30°C. Under these conditions, 4.5 g of product are obtained which shows the following quality pattern:
- content > 94%
- content of proteins < 0.3%
- mean molecular weight about 1,500,000 D
- transmittance of a 0.2% solution 99.8%

### EXAMPLE 15

As Example 14, in which the lyophilization takes place in vials instead of in bulk. Vials of type I neutral glass are used, on which stopper caps are placed. At the end of the operation, the vials are sealed in an inert gas atmosphere. The yield and the quality of the hyaluronic acid are perfectly equal to those of the product obtained according to the conditions in Example 14.

### EXAMPLE 16

The hyaluronic acid solution obtained according to the conditions of Example 12 is subjected to a lyophilization according to the conditions of Example 14. 4.5 g of hyaluronic acid having the following quality specifications:
- content > 93%
- content of proteins < 0.3%
- mean molecular weight about 1,500,000 D
- transmittance of a 0.2% solution > 99.8%

### EXAMPLE 17

The hyaluronic acid solution obtained according to the conditions of Example 12 is subjected to the action of a spray dryer. This gives a product of a quality equal to that of Example 16 in the same yield.

### EXAMPLE 18

A test is carried out under the conditions of Example 10, but with the exception that, after the purification on resins, the solution is fed to the microfiltration apparatus where it is concentrated to restore the initial volume, up to the time when the dry residue of the permeate disappears. The operation is continued until a concentration of 2% is reached, and then the solution is subjected to lyophilization under the conditions of Examples 14 and 15. At the end of the treatment, this gives 4 g of hyaluronic acid having the following quality specifications:
- content of dry product > 98%
- content of proteins about 0.1%
- transmittance > 99.8%
- molecular weight about 1,500,000 D

### EXAMPLE 19

1 g of soya lecithin, 0.5 g of cholesterol, 3.5 g of isopropyl myristate and 0.02 g of tocopherol acetate are dissolved in the minimum quantity of ethanol: this solution is dispersed with stirring in about 60 ml of an aqueous solution containing 0.1 g of disodium EDTA. 0.15 g of sodium methylparaben, 0.1 g of high-purity sodium hyaluronate, 2 g of vegetable glycol extracts, 7 g of propylene glycol and a sufficient quantity of carbowax to reach the desired viscosity are added to the liposomal dispersion thus obtained. The resulting gel, distributed in single-dose containers fitted with a cannula, proves suitable, thanks to the good tolerability, for the treatment of vaginal mucous membranes with pathological or dysmetabolic conditions, such as those affected by dryness.

### EXAMPLE 20

0.1 g of sodium hyaluronate is dissolved with stirring in about 90 ml of an aqueous solution containing 0.15 g of sodium methylparaben, 0.015 g of sodium propylparaben and 15 g of Poloxamer F127^{(R)}. 5 g of vegetable glycol extracts, 1 g of silicone derivative and 1 g of HPMC are added to the solution thus obtained. The solution is packed in suitable delivery containers which, without resorting to the use of propellants are able to generate a soft and persistent foam which, thanks to the good tolerability, can be used on the skin, even injured skin, or transferred onto the vaginal cavity by means of a cannula.

### EXAMPLE 21

0.2 g of high-purity sodium hyaluronate is dissolved with stirring in an aqueous solution containing 0.9 g of sodium chloride and as much phosphate buffer as necessary. The solution is made up to a final volume of 100 ml and packed under sterile conditions into single-dose containers suitable for instillation.

## Claims

1. Process for the preparation of hyaluronic acid, comprising the culturing, in submerged culture, of a microorganism of the genus Streptococcus zooepidemicus or Streptococcus equi, the inactivation of said microorganism and the isolation of said hyaluronic acid, characterized in that the culturing is carried out in the presence of divalent iron ions.

2. Process according to Claim 1, characterized in that the concentration of said divalent iron ions is within the range from 1 to 100 mg/l.

3. Process according to Claims 1-2, characterized in that said divalent iron ion can be provided by one of its salts selected from the group consisting of: sulphite, sulphate, chloride, fluoride, nitrate, chlorate and phosphate.

4. Process according to Claims 1-3, characterized in that the culturing is carried out using a strain of Streptococcus zooepidemicus deposited at CNCM on 21 July 1994 under the accession number I-1448.

5. Process according to Claims 1-4, characterized in that the additions of the carbon sources take place at various times during the fermentation cycle, namely:
a) a proportion of between 20% and 40% of the total at the moment of inoculation,
b) a proportion of between 20 and 40% of the total within 1 hour after the minimum level of dissolved oxygen has been reached, and
c) the remaining proportion within a period of 5-6 hours after the second addition.

6. Process according to Claims 1-5, characterized in that the pH of the culture broth is maintained within a pH interval of 6.5 ± 1.0 pH by addition of an alkali base, and that these additions are also effected during the phases of vegetative growth.

7. Process according to Claims 1-6, characterized in that, during all the growth phases of the microorganism, a overpressure of between 0.1 and 3.5 bar is maintained inside the fermenter.

8. Process according to Claims 1-7, characterized in that the sterilization of the complex raw materials of the growth media is carried out at a temperature below 120°C for a period of time of not more than 30 minutes.

9. Process according to Claims 1-8, characterized in that the following substances to the fermentation medium individually or in combinations of two or three can be added:
a) uracil in concentrations of between 0.01 and 10 g/l,
b) aspartic acid in concentrations of between 0.01 and 10 g/l, and
c) glutamine in concentrations of between 0.01 and 10 g/l.

10. Process according to Claims 1-9, characterized in that, at the end of the fermentation cycle, the microorganism is inactivated by the addition of hypophosphorous acid until a pH below 4.8 has been reached.

11. Process according to Claims 1-10, characterized in that the removal of the nutrient substances and complexes contained in the fermentation medium, and also of the soluble residues from the inactivation of the microorganisms, is effected in one or more microfiltration process stages which are carried out with orthogonal pressure or with a tangential flow upon a membrane or fibre or cartridges of ceramic or polymeric material or of sintered metal.

12. Process according to Claims 1-11, characterized in that the microfiltration process can also be used as a means for concentrating the solutions of hyaluronic acid or its salts.

13. Process according to Claims 1-12, characterized in that the porosity of the microfiltration structures is between 0.05 and 5 micrometres, preferably between 0.05 and 0.8 micrometres.

14. Process according to Claims 1-13, characterized in that the solution of hyaluronic acid and its salts is forced through a bed of ion exchange resin.

15. Process according to Claim 14, characterized in that the ion exchange takes place through the sequence of strong cationic resin followed by anionic resin.

16. Process according to Claim 15, characterized in that the treatment with the resins can be carried out in a column or in suspension.

17. Process according to Claims 1-16, characterized in that the process of extraction, or a part thereof, can be carried out in the presence of antiseptic substances directly added to the aqueous solutions which are to be treated.

18. Process according to Claim 17, characterized in that the substances having an antiseptic action are selected from the group comprising anionic, cationic or nonionic detergents, alcohols, aldehydes, ketones, phenols and phenolic derivatives.

19. Process according to Claims 1-18, characterized in that the recovery of the solid product can take place directly from the culture broth solutions without any isolation therefrom after drying, which occurs by the processes of lyophilization, atomization or exposure to heat under reduced pressure.

20. Hyaluronic acid of high purity, free of β-haemolysin, obtainable by the process of Claims 1-19.

21. Streptococcus zooepidemicus deposited at CNCM on 21 July 1994 under the accession number I-1448.
